# EUROPEAN PATENT APPLICATION

(11) **EP 1 543 841 A1**
(43) Date of publication of application: **22.06.2005**
(21) Application number: 03793565.7
(22) Date of filing: 13.08.2003
(51) Int. Cl.: A61K 47/40, A61K 47/24, A61K 9/14, A61K 9/127, A61K 9/10, A61K 31/337

(54) **SOILD NANO PHARMACEUTICAL FORMULATION AND PREPARATION METHOD THEREOF**

(30) Priority: 15.08.2002 CN 02128845; 25.11.2002 CN 02149146
(71) Applicant: Liu, Yunqing, Haidan District, Beijing 100083 (CN); Liu, Xiying, Haidan District, Beijing 100083 (CN); Liu, Wei, Haidan District, Beijing 100083 (CN); Liu, Tong, Haidan District, Beijing 100083 (CN)
(72) Inventor: Liu, Yunqing, Haidan District, Beijing 100083 (CN); Liu, Xiying, Haidan District, Beijing 100083 (CN); Liu, Wei, Haidan District, Beijing 100083 (CN); Liu, Tong, Haidan District, Beijing 100083 (CN)
(74) Representative: Goddar, Heinz J., Dr.
(86) International application number: PCT/CN2003/000663
(87) International publication number: WO 2004/022100

(57) **Abstract**

Disclosed is a method of preparing low water-soluble medicine into solid nanometer pharmaceutical formulation. According to the characters of molecular aggregates such as supramolecular chemical micelles and vesicles, the formulation, which based on the hydroxypropyl-beta-cyclodextrin and phospholipid, is prepared under the condition of hyperthermia sterilization and decompression. Such nanometer formulation is sterile particle or powder with loose porosity. For directly intravenous use, the formulation has targeting activity, sustained release and long circulating characters. While as a solid oral product, it is fast-release, fast-effective, and improved bioavailability characters, and is readily melted in mouth. The formulation utilizes secure accessories, traditional equipments and methods, thus, it is suited to be used and manufactured widely. Also disclosed is intravenous formulation of anticancer paclitaxel, which **characterized** that there has no polyoxyethylenated castor oil in it. Such intravenous formulation is nonallergic so that it has higher security and efficiency compared to present commercially available paclitaxel formulations.

## Description

### Field of the Invention

The present invention relates to a solid nanometer medicine and the method of preparing the same, and more particularly to preparing the solid nanometer medicine by complexing sparingly-soluble organic medicines and one amphipathic matrix (carrier). The invention further relates nanometer medicines of paclitaxel for injection and the method of preparing thereof.

### Background of the Invention

Sparingly-soluble medicines and their preparations, which are prepared by conventional methods, have low and alterable bioavailability to effect the medicine efficacy. For the new active compounds, the preparations prepared by conventional methods may affect the result of filtration before clinic application and clinical trial, and may even make the filtration impossible. It is reported that about 40 percent of active substances filtrated by assembly chemistry are sparingly-soluble in water, which is similar to the ratio of the sparingly-soluble medicines used in clinic. It is understood that increasing the solubility of water-insoluble or sparingly-soluble medicines is important.

In recent years, nanometer technology has developed, in addition to the methods of preparing aqueous soluble complex. The technology highly disperses medicines to steady submicro-granules (nanometer crystals) by physical or physical chemical methods, which render many new characteristics to medicines, such as higher solubility, biological availability, medicine efficacy, target aiming , extending release time and medicine efficacy. These medicines can be used to process medicines used in or out of the gastrointestinal tract, especially for intravenous injections etc. This technology has promising prospects.

At present the success of nanometer technology depends on the efficiency of the preparation technology, the possibility of mass production and the cost of production, especially on the security of the used carriers and the types of preparations. As for the manufacture technology, one of the mainly used methods is trituration, for example the NanoCrystal technology of Elan (preparing the nanometer crystal with stabilizers). Others are the super-critical fluid extraction technology and other derivative technologies. Based upon NanoCrystal technology, sirolimus (immunodepression), a tablet produced by Elan, came into the market in 2001. This is the first sold nanometer medicine. There are some other medicines in development. The above-described methods need special equipment and technologies, which are a hindrance to the common pharmaceutical enterprises.

The nanometer medicines prepared by the conventionally pharmaceutical methods using excipients as the carriers are under the research at present, for the carriers and preparing methods do not adapt to demands of clinic and industry manufacture.

The solubility in water of the famous antineoplastic paclitaxel is 0.006 mg/ml. In fact, it doesn't dissolve in water. Therefore, the pivotal technology of the intravenous injection is increasing its solubility in water. The present clinical Chinese agents Zisu and Taisu, American agent Taxol® (BMS of the U.S.A), and Anzatax® (Fauliding of Australia) are prepared to the solutions of 30mg/5ml with the solvent of polyoxyethelene castor oil (Cremophor EL surfactant) and anhydrous alcohol (v/v 1:1). They dissolve in water and steady in 8 to 12 hours (no crystal separate out). They are diluted with solvents for injection (for example 5% glucose) before using. However, these solvents have some problems as follows:
1. Polyoxyethelene castor oil may improve the release of histamine to high anaphylaxis. Before taking the agents, antihistamine (oral medicines and injections) must be administrated under the strict observation.
2. Polyoxyethelene castor oil can extract dioctyl phthalate, which is the general plasticizer in polyvinyl chloride infusion apparatus, to increase the toxicity of medicine solution. Therefore, it is necessary to use polythene and glass infusion apparatus.
   Because these problems increased patients' pain and cost of medical treatment, a solution is desired as soon as possible. Therefore, Paclitaxel preparation for intravenous injection without polyoxyethelene castor oil has been a research hotspot. In recent years pharmacologists all over the world have done a great deal researches, for example "Review on Paclitaxel for intravenous injection without Polyoxyethelene castor oil, Xiaohui Wei *et. al*. Chinese Journal Pharmaceuticals 2001.32(4):188". It is reported that the types have been researched including intravenous injection, general emulsion for intravenous injection, submicro-emulsion for intravenous injection and micro-emulsion for intravenous injection, micro-particle and polyethylene glycol derivates of Paclitaxel. The preparations with the carrier of protein showed obvious progress. However, most of the preparations are not up to the demands of medicine security and industry manufacture. There is no report about their applications and sales.

Lately an aqueous soluble intravenous injection with the carrier of polyglutamic acid has undergone clinic test of Stage II in America, and Stage I in Britain (please refer to Scrip 2001(2690)14). Acusphere Corporation has a particular formulation of Paclitaxel in clinic test, which belongs to the Hydrophobic Medicines Delivery System (HDDS) (please refer to information on Internet on May 30th, 2002). A new formulation of paclitaxel, Genexol®-PM (1)of Sanyo of Korea, has been permitted to the clinic test of Stage I by FDA (please refer to Scrip 2002(2734/35)31). The above reports show researches have achieved obvious developments all around the world. These preparations mostly belong to the nanometer preparation.

Domestic research is carried out in the laboratories which are far from the production or clinic in view of carriers and preparation technology (please refer to "the preparation and research in vivo and in vitro of the long circulated solid lipide nanometer granule of Paclitaxel, Dabing Chen *et. al*. Acta Pharmaceutical Sinica 2002,37(1):54 ~ 58"). The updated technology, which applies the nanometer preparation of sparingly-soluble medicines to intestinal tract or not, is the hotspot of researching the preparation of medicine preparation research. Its success of production and clinic application depends on the security and easy-selection of the carrier and the popularity of its technology, equipment and method.

An aspect of the invention lies in providing a solid nanometer medicine and the method of preparing the same. The invention prepares the solid nanometer granules of medicine by using amphipathic matrix under conventional conditions, so the invention satisfies the clinic requirement. The invention can be used for preparing the nanometer granules of sparingly-soluble or water-insoluble medicines in common conditions, especially for preparing the aqueous soluble nanometer granules of the sparingly-soluble Paclitaxel.

### Summary of the Invention

The invention provides a method of preparing solid nanometer medicine, comprising reacting the medicine with amphiphiles, producing the complex of the amphiphiles and medicine, preparing the sterile solid nanometer medicine by concentrating, solidifying and inflateing multihole solid.

The invention also provides a method of preparing medicine complex, comprising providing an amphipathic system, reacting the medicine with amphiphiles, producing the complex of the amphiphiles and medicine.

The invention further provides solid nanometer granule of Paclitaxel. These granules are soluble in water. It can be prepared as an injection, micro-emulsion and submicro-emulsion.

### Description of the Invention

### 1. Definitions

The term of "amphiphile" used in this specification means a substance having both hydrophilicity and liposomeity. In the invention, the preferred amphiphile includes hydroxypropyl-beta-cyclodextrin (HP-β-CD) and phospholipids.

The term of "micelle" means a microstructure of hydroxypropyl-beta-cyclodextrin (HP-β-CD) dissolved in an aqueous solution, in which the interior is hydrophobic and the exterior is hydrophilic, and the size ranges from 1 to 10 nm.

The term of "vesicle" means a microstructure formed by phospholipids in water, which is also called microvesicle or liposome, in which the core is filled with water and the periphery is liposome. The core diameter can range from 20 to 30 nm, decades even up to hundreds of nanometers in different cases. The nanometer dimension of pharmaceutical preparations is usually from 1 to 1000 nm.

The term of "complexes" means a coordination compound which is formed by hydroxypropyl-beta-cyclodextrin (HP-β-CD), phospholipids mentioned as above and compounds (active matters such as medicine) bonding each other with weak bonds. It can also be regarded as a complex with special space structure composed of two or more matters. "Complexes" used as herein doesn't restrict the form of acting force or the manner of interaction, and It is solely used to describe a steady co-existence state of two or more substances after a certain interaction. In the invention, the term "complexes" especially refers to the complexes, which are formed by medicines or compounds with pharmaceutical prospect in the microstructure of a micelle, vesicle etc., wherein the medicines or compounds with pharmaceutical prospect locate in the interior of micelle, vesicle etc., existing in a relatively close circumstance, while the whole complex entirely interacts with the environment with the amphiphiles composing micelle or vesicle. For the convenience of description, "medicines or compounds with pharmaceutical prospect" are briefly referred to as "medicines". It is to be understood for one in the art that the method of the invention can deal with any compound, which needs this treatment, especially for dealing with insoluble organic compounds, especially for water-insoluble organic compounds.

The term of "concentration" means a conventional technique in the art, reducing or removing the solvent from the solution system comprising the complexes and obtaining the complexes.

The term of "solidifying" means a process in which substances composing micelle or vesicle in the above complexes becomes relatively compact. In solidifying, chemical action may take place, such as weak bonding among molecules, and additionaly or alternatively physical action takes place. Inflating takes place during solidifying, reducing the pressure when heating: solvent is rapidly evaporated to foam, forming porous loosen solid, which is water-soluble, and having a similar effect with freeze-drying.

The nanometer medicines of the invention can be illustrated by the principles of supramolecular chemistry. In specific acts, they comprise that hydroxypropyl-beta -cyclodextrin (HP-β-CD) forms micelles in a solution (the micelle is hydrophobic in the interior and hydrophilic in the exterior, and its size ranges from 0.5 to 10 nm), phospholipids form vesicles in water (microvesicle-liposome), the core of the vesicle is filled with water, and the exterior is liposome, and the diameter of the core ranges from 20 to 30 nm, tens up to hundreds of nanometers), and that forms a multiple steady amphipathic (hydrophilic, liposome) system, since both micelle and vesicle can contain medicine molecules to form a complex, in accordance with the principles of molecular recognition and self-organization, combining medicines in the system, and medicines exist thereof in molecular state.

In one embodiment of the invention, using hydroxypropyl-beta -cyclodextrins (HP-β-CD) and phospholipids as a matrix, medicines or compounds with pharmaceutical prospect, especially insoluble or sparingly-soluble medicines or compounds with pharmaceutical prospect are prepared to a complex incorporating with the matrix, and the particle diameter of the complex is small, and achieves the nanometer level in pharmacy.

The amphipathic system of the invention is characterized by the microcosmic characteristic of both hydrophilia and lipophilia. I.e. the amphipathic substance is both hydrophilic and liposome. Thus, the above-described two substances are only examples, and the invention does not restrict specific substances used. One skilled in the art can choose other substances with these properties according to the spirit and esence of the invention under the description of the invention, and these are included in the invention. The microcosmic configuration of these substances in water, for example micelle and vesicle, can vary with conditions, and this varying is in the mind of one in the art.

The amphipathic system can include one solute, such as an aqueous solution of hydroxypropyl-beta-cyclodextrin (HP-β-CD); and can also include two solutes, such as an aqueous solution of HP-β-CD and phospholipids; and can further include more solutes, that is an aqueous solution adding other substances with similar properties with hydroxypropyl-beta-cyclodextrin (HP-β-CD) and/or phospholipids. The amphipathic system used herein is a system comprising amphipathic substances. The amphiphile is a substance having both hydrophilia and lipophilia, which can form a certain microstructure in water or a hydrophilic organic solvent, such as micelle and vesicle. These microstructures can be regarded as a micro-container.

Furthermore, for facilitating the amalgamation in the microstructures of different amphipathic solutes in an aqueous solution, a suitable surfactant may be added. For example surfactant Tween® 80 can be added into the aqueous solution of hydroxypropyl-beta-cyclodextrin (HP-β-CD) and phospholipids to facilitate the amalgamation of micelles and vesicles formed by them in the aqueous solution.

The solvent of the amphipathic system of the invention is preferably water. Other hydrophilic organic solvents can also be used, such as ethanol. The hydrophilic organic solvent of the invention may be selected from the group consisted of lower fatty acid ester solvents, hydrocarbon solvents, halogenated hydrocarbon solvents, furan solvents, acidamide solvents, lower fat alcohol solvents, nitrile solvents, ketone solvents and mixtures thereof. However, the existence of water is necessary. In an embodiment of the invention, the above-described organic solvent is selected from the group consisted of methyl acetate, ethyl acetate, butyl acetate, petroleum ether, cyclohexane , dichloromethane , chloroform , tetrahydrofuran , dimethyl acetamide, dimethyl formamide, methanol, ethanol, propanol, butanol, acetonitrile, acetone and mixtures thereof.

When the amphipathic system of the invention is prepared, the selection criterion of solvent is whether the solute can form a certain microstruture in a suitable solvent, such as the above-described micelle and vesicle. In a preferred embodiment of the invention, the solute used is hydroxypropyl-beta-cyclodextrin (HP-β-CD) and phospholipids; and the solvent is water.

In preparation of medicines of the invention, the solute in the amphipathic system of the invention may function as a carrier of medicines, and known as "matrix" or "carrier" too. Therefore, "the matrix formulation" in the invention has the same meaning, for representing the solute formulation in the amphipathic system of the invention. In the preparation of the invention, surfactants etc. can be used, thus "the matrix formulation" can further include surfactants and stabilizers.

In one aspect of the invention, the specific matrix formulation involves using 100 % of hydroxypropyl-beta-cyclodextrins (HP-β-CD). In another aspect of the invention, the specific matrix formulation involves using hydroxypropyl-beta-cyclodextrins (HP-β-CD) and phospholipids, wherein the ratio of them is 1:0.05~0.3.

Other amphipathic solutes, one or more surfactants, one or more stabilizers can be added into the matrix formulation in accordance with various cases. One effect of these surfactants is to facilitate interactions such as amalgamation between microstructures formed by solutes in the amphipathic system of the invention etc., thus using similar substances instead of said surfactant herein also falls within the scope of the invention. Certainly, these additional components can be not added into the matrix formulation, but used in other acts.

The preparation of the amphipathic system of the invention is a dissolving process in substance.

According to the specific properties of object medicines or compounds with pharmaceutical prospect, the corresponding solvent and above-described solute are selected, and dissolved to prepare the amphipathic system for the object substance. The dissolution is carried out at temperature from 30 to 100°C, preferably from 60 to 75°C. The solute species and the dissolving sequence in the matrix formulation can have kinds of varieties, and solvent can have kinds of choices, but only need satisfy forming the microstructure required in solution, such as microvesicle or micelle, after dissolving. Certainly operations of stirring, pH adjustment etc during dissolving can also be carried out according to particular demands.

The microstructures of micelle, vesicle etc in the amphipathic system can contain medicine molecules, and form a complex with the medicine molecule. The invention combines medicines into the system according to the principles of molecular recognition and self-organization, wherein medicines exist in a molecular state.

In a preferred embodiment of the invention, the matrix coordinated by hydroxypropyl-beta-cyclodextrins (HP-β-CD) and phospholipids are water-soluble. They dissolve in water and become transparent solution or microemulsion. When medicines are added thereto, the medicine is contained into micelles formed by hydroxypropyl-beta-cyclodextrins (HP-β-CD) and microvesicles formed by phospholipids.

The action between medicines and the amphipathic system owes to the interaction between molecules. As mentioned above, the amphipathic system of this invention is a special liquid system comprising microstructures of micelles and vesicles. The difference between hydrophilic and liposome properties of the interior and exterior of the microstructures, and the volume size of these microstructures themselves, is the matrix of which they can valuable disperse sparingly-soluble substances in water comprising medicines and compounds with clinical prospect. One aspect of the invention is to prepare a new form of prior medicines, i.e. solid nanometer medicines. Another aspect of the invention is to prevent compounds with clinical prospect from being selected with pretermission and mistake, thus in the invention "medicine" should be understood in a broad sense, and generally refers to substance required to increase the solubility. Thus, the use of the methods of the invention is not limited to the field of pharmaceutics and medicines research.

Medicines are added into the amphipathic system. Medicines are contained in the microstructure of the amphipathic system by the interaction between molecules. Such suspension can supply the market after routine disposal such as separate packing. The microstructure of the amphipathic system effectively realizes the dispersion of medicines.

Furthermore, the amphipathic system comprising medicines or object substances may acquire solid granules substances by a certain process. For example, the matrix coordinated by hydroxypropyl-beta-cyclodextrin and phospholipids is water-soluble, which dissolves in water and forms a transparent solution or microemulsion. When medicines are added therein, the medicines are contained in micelles formed by hydroxypropyl-beta-cyclodextrin and microvesicles formed by phospholipid. It is a transparent solution in a liquid state, and after decompressing and concentrating at heating, it solidifies through a transparent or semitransparent glassy state, and presents a loose porous state after swell-drying. If using hydrophilic organic solvent to dissolve hydroxypropyl-beta-cyclodextrins (HP-β-CD) and phospholipids, the variety does not have an apparent difference. In the invention, the solution having a microstructure comprising medicines thereof is concentrated, a complex with weak bonding between molecules forms between hydroxypropyl-beta-cyclodextrin and phospholipids, therefore forms a compound order coating medicines.

The loose porous substance after swell-drying can be prepared into loosen porous sterile solid granules or powders. Such granules or powders dissolves at once in water and forms a microemulsion or submicroemulsion. Because both hydroxypropyl-beta-cyclodextrin and phospholipids have a property of preventing medicine molecules from aggregating in water, medicine particles corresponding steadily suspends in a solution, wherein the diameter of the medicine particles ranges from 1 nm to about 300 nm, preferably from 30 nm to about 300 nm, and more preferably from 50 nm to about 200 nm, and the average diameter ranges from 100 nm to 200 nm. If a stabilizer is added, the effect can be improved.

Transmission electron microscope measurement shows that the particle diameter distribution ranges from 1 to 300 nm, and it is correspondingly steady in several hours, days or longer period. The index of screening and evaluating the matrix formulation is carrying capacity; particle diameter and distribution of particle diameter; and suspending stability etc.

The method of the invention can be used for various medicines, for example, but not to be limited:
- A.: Paclitaxel (Taxol®) preparation;
- B.: Artemether preparation;
- C.: Dihydroartemisinin preparation
- D.: Busulfan preparation;
- E.: Nimodipine preparation;
- F.: Nitrendipine preparation;
- G.: Nifedipine preparation;
- H.: Diazepam preparation;
- I.: Cinnarizine preparation;
- J.: Lovastatine preparation;
- K.: Simvastatine preparation.

In the invention, phospholipids include various phospholipids, such as soybean lecithin.

The surfactant can be Tween® 80, O/W- type, and the amount is determined by experiments for different medicines.

The stabilizer can be polyvidone K₃₀, K₁₅ (PVP K₃₀, K₁₅) or dextran 40, 70 etc and the amount is determined by experiments.

The nanometer medicines of the invention have a fast-releasing property, and the tablet prepared is an oral thawing tablet, it releases rapidly and effectively; when the nanometer medicines are used for intravenous injection, it also shows long-circulated and targeted etc.

The technique of the invention can employ conventional pharmaceutical equipment. It can make a commercial and high-efficient production. The properties of the product are steady, which can be directly or secondarily processing to prepare various injecting or oral preparations and it is a unique and universal method with lower cost to prepare nanometer medicines.

The specific examples in which loosen porous germ-free granules or powders are processed directly or secondarily to preparation:
1. Directly split charging and prepare powers for injection;
2. Adding accessories and prepare various oral preparations;
3. Prepare various liquid preparations.
   Particularly, it is an aspect of the invention to provide a method by which indissoluble organic compounds are prepared to a new pattern of solid nanometer medicine according to principles of supramolecular chemistry above-described. The method remarkably improves the solubility and dissolution rates of the indissoluble organic medicines in water, and has targeted property and a certain extent of slow-releasing and prolongation effort to stabilize and increase medicine effect. It is available to directly split charging and prepare injection, and to secondarily process and prepare oral tablet, capsule/ granule and other dosage forms. The invention employs common safe accessories and conventional equipment, and is an extensively suitable method of preparation with high efficiency and low cost.

In a specific technical solution, a matrix and surfactants are used; in another specific technical solution, matrix, surfactants and stabilizers are used. The surfactants and stabilizers are selectivity added according to the specific situation. The matrix and relative reagents employed are physiologically compatible, and are proven safe and reliably by years of clinic use. The diameter of medicine particles can be controlled by adjusting the matrix formulation, to fit the requirement of various medicine target organization. Since the matrix contains polyhydroxy, it has a character of long-circulation, i.e. recessive when it is used for intravenous injection that is proven by references and experiments.

The main procedure of the method of the invention is shown as below: at the temperature from 30 to 100°C and in a clean condition (10,000 level), a matrix is dissolved in water or a hydrophilic organic solvent, then medicines are added, if necessary surfactants and stabilizers can be added; adjust pH if necessary, stir to dissolve them completely. Decolourize, de-pyrogen, filter to remove bacterium, and the cleaning solution is concentrated under decompression when heating, solidifid, swelled, and dried. Discharge, and crush products to granules or powders for preparation.

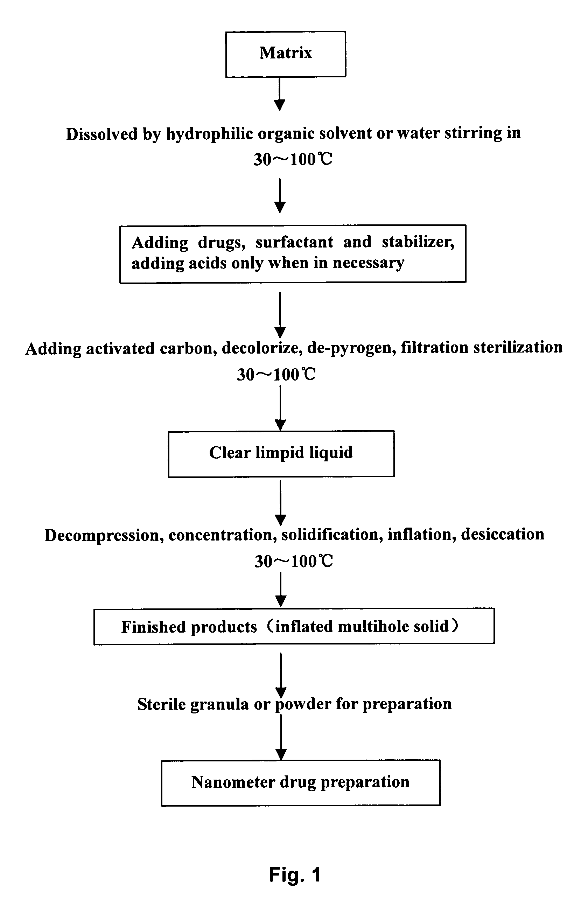

Now refer to Figure 1, the method of the invention is described in detail. Firstly, the matrix is dissolved in a hydrophilic organic solvent or water. The matrix can be hydroxypropyl-beta-cyclodextrin, or other compositions containing hydroxypropyl-beta-cyclodextrin, such as a composition of hydroxypropyl-beta-cyclodextrin and phospholipids. Each ingredient of the matrix can be added into a solvent simultaneously or successively. The solvent may be water or other suitable organic solvents. The process dissolving different matrixes are not the same. Generally the temperature when dissolving is 30 to 100°C, preferably 60 to 75°C, and more preferably 60 to 70°C. For facilitating dissolution, stirring can be employed, wherein the requirement for conditions, such as the cleaning condition (10,000level), should depend on each instance. Heating can also depend on the solute.

Then to the solution dissolving the matrix, medicine is added and a complex with a microstructure of the medicine and matrix is formed in the solution. From microcosmic view, the matrix dissolves in an organic solvent or water and forms a microstructure such as micelle, vesicle etc, and the medicine forms a complex through entering the microstructure. The formation of the complex has changed the physical character of the medicine, and achieves an aspect of the invention. Therefore, one aspect of the invention is to prepare a medicine complex. Microstructures such as micelle, vesicle etc. can be regarded as a special carrier of the invention for preparing medicines. However, these microstructures have the currency, and it is apparent to one in the art that other medicines, which are not mentioned by the invention, can also employ this carrier. Thus one aspect of the invention is to provide a special carrier for prepare a medicine. Substances and methods, which can form such a microstructure, are not limited in those, which are definitely illustrated by the invention. It is a major idea of the invention that supermolecules are used for changing properties of medicines, and any practice using this idea can be regarded as utilizing the invention.

In the invention, surfactants and stabilizers can also be employed. These surfactants can be added after matrix dissolving, or added together with the medicine, and the function is to facilitate the interaction between microstructures such as micelle etc. The stabilizer is added after the medicine is put into the matrix solution in order to stabilize the supramolecular complex is preferred. The acid or alkali may be added if necessary.

In the invention, the formed complex is a supramolecular order. Because the medicine is coated in the microstructures of hydroxypropyl-beta-cyclodextrin, phospholipid and other substances, many medicines may be modified by such method. In the invention, modification is applied in a wider field. The water-solubility and bioavailability of the modified medicine depend on the coating material. The medicine stays inside comparatively unattached, kept from the direct action of the outside circumstance. Furthermore, after the medicine is coated, we can make the molecules combined or having physical-chemical hanges, even antibody binding to the microstructures surface in order to send special medicine to the definite spot.

The solid nanometer medicine complex is obtained from the solution containing the supramolecular order by concentration at a low pressure. The temperature for concentration at a low pressure is 30~100°C, 60~75°C is preferred and 60~70°C is more preferred. Concentration at a low pressure promotes the interaction between hydroxypropyl-beta-cyclodextrin and phospholipid, which forms a comparatively compact structure coating outside the supramolecular order mentioned above. The medicine stays inside comparatively unattached, and stay in multi-forms, such as liquid, half liquid, solid, half solid, crystalloid, mixture, and solution. Therefore, the medicines of the invention exist in especially physical state: one or more amphipathic pellicles or coatings are outside, medicine or substance containing medicine is inside. The character of the medicine complexes of the invention is that the granules' diameter is from about 1 nm to about 300 nm, about 30 nm to about 300 nm is preferred, and about 50 nm to about 200 nm is more preferred. The average diameter is 100~200 nm.

After concentration at a low pressure, the surface matter of complex forms a comparatively compact structure. Such complexes can be viewed as solid granules. The forms of medicines within the complex differ, such as liquid state as described-above. Therefore, the solid nanometer medicines are named from their appearance and granule size. In fact, the surface substances of the complex, such as hydroxypropyl-beta-cyclodextrin, phospholipids, etc. may be not the solid state. Actually, their structures are comparatively compact and in order, this kind of microstructure, such as liquid crystal state, may contain medicines. So the invention emphasizes the difference between the medicine and the outside coating, and the fact that such difference has changed the dissolution quality of the inside medicine.

One aspect of the invention is the discovery of the microstructure containing medicines.

In the invention, concentration at a low pressure can be substituted by other methods such as spray dying. Any way is available which can stabilize the complex, such as ultrasonic disposal, stirring acutely. There are many methods to transform the complex into solid granules. We also can use these methods together. The solvent evaporated may be used twice if it equipped with a condensation system.

The solidification of the complex mainly occurs on its surface, for example, the solidification of hydroxypropyl-beta-cyclodextrin and phospholipid. Coated by them, the medicine stays inside comparatively unattached. Such substances may transform into inflated multihole solid by inflating and dehydrating disposal. The solid exists in powder or granule form.

Coated by the amphiphile, the medicine granules or liquid drops are suitable for many preparations for clinic, such as agent for oral administration or injection.
The term "nanometer" used herein is the quantum grade, which means the granule diameter is less than 1000 nanometers. Therefore, from the view of pharmacy, the size of the complex of the invention is on the nanometer level.

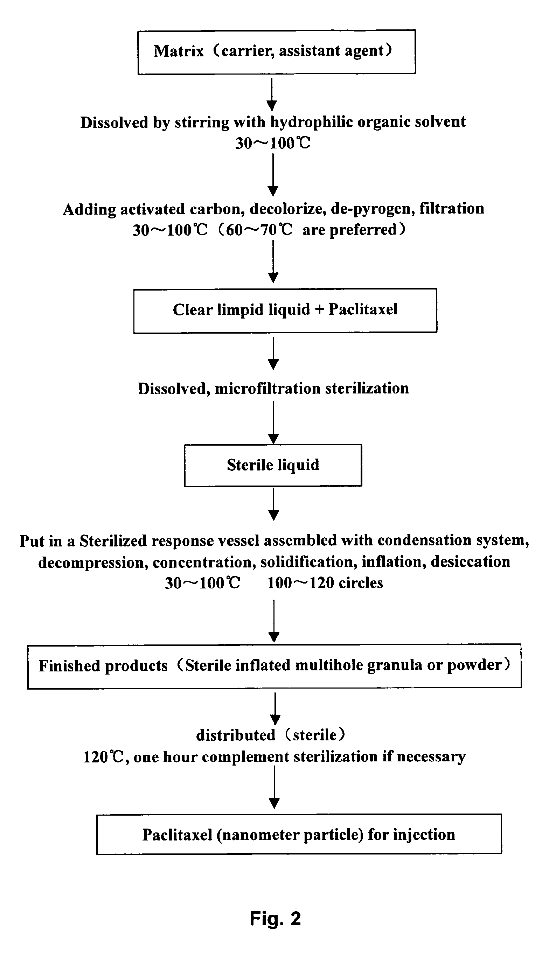

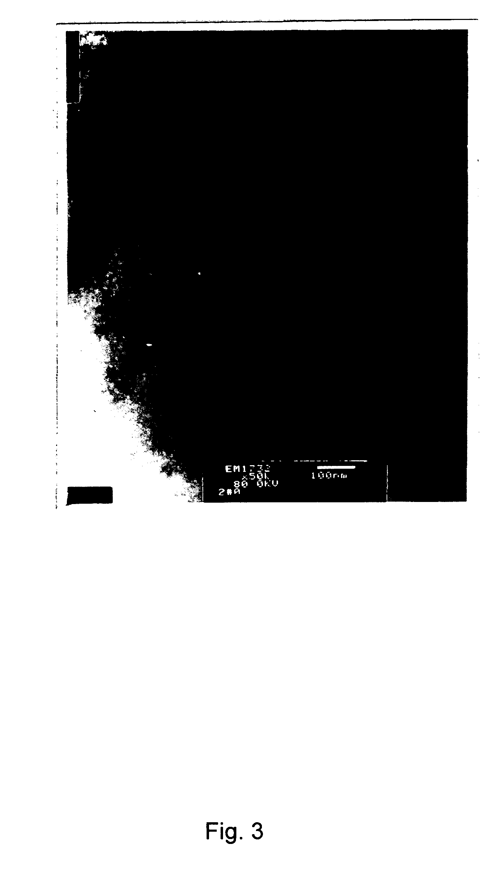

In one example, we prepare the paclitaxel for injection (NanoCrystal), which has no polyoxyethelene castor oil to introduce anaphylactic disease. Referring to Fig. 2, we put hydroxypropyl-beta-cyclodextrin into water to get micelle, and put phospholipid into water to get vesicle. By applying the proper surfactant, the two characters are involved in a steady multi-amphipathic system in the aqueous solution. The homogeneous system comes into being after the molecular medicine combines with it. The sterile loose granules or powder appears after concentration and dying. Fig. 3 is the transmission electron microscope picture of the diameter of Paclitaxel particles for injection (in water) (X50000). It is apparent that the most particles' diameter is shorter than 50 nm. Such granules or powder can dissolve in water rapidly. Because the solubility in water is smaller than in the preparation procedure, the separated medicines form into emulsion or submicro-emulsion. The medicine effect test of the Paclitaxel of the invention (Paclitaxel nanometer particle) is anti-neoplasm. We select three neoplasm cells: Hepatoma (H22), sarcom (S-180), lung cancer (Lewis Lung Cancer, LLC) to inoculate to ICR SP and C57BU6 SP mice. On the next day do the medicine effect test, depressing neoplasm test. Comparing with the clinic Paclitaxel injection (Beijing Xie-he Pharmaceutical Factory, production batch No. 020202), the test proves that the effect is better than the preparation from market. Intravenous injection, intraperitoneal injection and intragastric administration are according to the clinic dosage and concentration.

The LD₅₀ of two preparation mouse acute toxicity tests are 84 .73 mg/kg and 84.55 mg/kg respectively, the difference is not significant.

### Description of drawing

Fig. 1 is the technological flow sheet of the method of the invention.
Fig. 2 is the technological flow sheet of paclitaxel for injection.
Fig. 3 is the transmission electron microscope picture of the diameter of paclitaxel particle for injection (in water) (X50000). It is apparent that the most particles' diameter is shorter than 50 nm.

### Preferred Embodiments of the Invention

The present invention is more clearly understood from the following detailed description taken in conjunction with the accompanying drawings. However, it should be recognized that various modifications, additions and substitutions are possible, without departing from the scope and spirit of the invention as disclosed in the accompanying claims. The examples are only for the purpose of description, not the limitation to the invention.

### Example 1 Formulations of starting material

| 1. Paclitaxel for injection Specification: 30mg/2.5g | | |
|---|---|---|
| Matrix formulationtion | hydroxypropyl-beta-cyclodex trin | 60 |
| | Phospholipid | 8 |
| | Tween® 80 | 9 |
| Chief parameter: | Dosage% | 1.2 |

| 2. Artemether for injection Specification: 60mg/2.2g | | |
|---|---|---|
| Matrix formulation | hydroxypropyl-beta-cyclodex trin | 31.5 |
| | Phospholipid | 3 |
| | Tween® 80 | 1.5 |
| Chief parameter | Dosage% | 2.7 |

| 3. Dihydroartemisinin for injection Specification: 40mg/1.5g | | |
|---|---|---|
| Matrix formulation | hydroxypropyl-beta-cyclodex trin | 31.5 |
| | Phospholipid | 3 |
| | Tween® 80 | 3 |
| Chief parameter | Dosage% | 2.7 |

| 4. Busulfan for injection Specification: 2mg | | |
|---|---|---|
| Matrix formulation | hydroxypropyl-beta-cyclodex trin | 17 |
| | Phospholipid | 1.7 |
| | Tween® 80 | 0.75 |
| Chief parameter | Dosage% | 5.1 |

| 5. Nimodipine for injection Specification: 12mg/0.9g | | |
|---|---|---|
| Matrix formulation | hydroxypropyl-beta-cyclodex trin | 64 |
| | Phospholipid | 3.5 |
| | Tween® 80 | 5 |
| Chief parameter | Dosage% | 1.3 |

| 6. Nimodipine for oral administration Specification: 20mg/tablet | | |
|---|---|---|
| Matrix formulation | hydroxypropyl-beta-cyclodex trin | 7.5 |
| | Phospholipid | 1.0 |
| | Citromalic acid | 0.5 |
| | Tween® 80 | 1.0 |
| Chief parameter | Dosage% | 10 |

| 7. Nitrendipine for oral administration Specification: 10mg/tablet | | |
|---|---|---|
| Matrix formulation | hydroxypropyl-beta-cyclodex trin | 13.5 |
| | Phospholipid | 1.0 |
| | Citromalic acid | 0.5 |
| | Tween® 80 | 1.0 |
| Chief parameter | Dosage% | 6.3 |

| 8. Diazeperm for injection Specification : 10mg/0.1g | | |
|---|---|---|
| Matrix formulation | hydroxypropyl-beta-cyclodex trin | 8 |
| | Phospholipid | 1 |
| Chief parameter | Dosage % | 11 |

| 9. Cinnarizine for injection Specification: 20mg/0.22g | | |
|---|---|---|
| Matrix formulation | hydroxypropyl-beta-cyclodex trin | 8 |
| | Phospholipid | 1 |
| | Tween® 80 | 0.5 |
| Chief parameter | Dosage% | 10.5 |

| 10. Nifedipine Specification: 5, 10, 20mg/ tablet | | |
|---|---|---|
| Matrix formulation | hydroxypropyl-beta-cyclodex trin | 8 |
| | Phospholipid | 1.0 |
| | Polyvidone | 2 |
| | Tween® 80 | 2 |
| Chief parameter | Dosage% | 7.7 |

| 11. Lovastatine | | |
|---|---|---|
| Matrix formulation | hydroxypropyl-beta-cyclodex trin | 36.5 |
| | Phospholipid | 1.0 |
| | Tween® 80 | 1.5 |
| Chief parameter | Dosage% | 2.5 |

| 12. Simvastatin | | |
|---|---|---|
| Matrix formulation | hydroxypropyl-beta-cyclodex trin | 36.5 |
| | Phospholipid | 1.0 |
| | Tween® 80 | 1.5 |
| Chief parameter | Dosage% | 2.5 |

### Example 2

### The preparation of paclitaxel for injection

In preparation, the starting materials are hydroxypropyl-beta-cyclodextrin , soya phospholipid-80 (Tween® 80) as assistant agent; Polydone K (PVP K); and low-molecilar dextran, etc.

| The formulation of preparing paclitaxel (nanometer particle) for injection 30mg/2.5g (ampule) | |
|---|---|
| Paclitaxel | 1g |
| hydroxypropyl-beta-cyclodextrin (for injection) | 60g |
| Phospholipid (for injection) | 8g |
| Polydone K | 5g |
| Polysorbate-80 | 9g |
| | 30mg/2.5g (ampule) |

According to the general measure, the dosage % in the invention is 1.19.

To the demand of applications, we can adjust the ratio of matrix and assistant agent in the formulation to change the solubility of medicines and control the diameters of the medicine particles.

The matrix and assistant agent in the formulation are biocompatible and safe. Furthermore, we can easily buy them on the market. Polyvidone is suspending stabilizer, polysorbate-80 is surfactant O/W.

The process of preparation is as following:
Under clean conditions, the matrix is dissolved in hydrophilic organic solvent according to the formulation, added the assistant agent, stirred to dissolve completely, then warm-up to 30~100°C. Next, activated carbon is added into the obtained solution to decolorize and de-pyrogen, filtrated. Paclitaxel is added into the clear liquid, and then sterilized by filtration after dissolving. The filtrate is put into a circumvolving response vessel assembled with condensation and solvent retrieving system at 30~100°C(60~70°C is preferred), 100~120circles, decompressed and concentrated under low pressure, solidified, inflated and solidified again. Then dried under low pressure for 2~3 hours. The obtained products are crushed into particles or power, then directly distributed into powder for injection. The production ratio is more than 98%.

### Characters of obtained products:

1. Property
The products are amorphous white or near white granules or powder, easily damp. No odor or tiny smell of Soya (smell of Soya phospholipid) is felt.
2. Solubility
Dissolved in diluted ethanol, the products present clear liquid. Dissolved in water, the products would separate out particles, self-emulsified to form emulsion liquid or submicro-emulsion liquid, which is suspending uniformly and comparatively stabilizing.
3. The particle diameter and its distribution
The product is homogeneous system during the preparing, however particles may appear due to reducing solubility in water(or solvent for injection). At the same time, hydroxypropyl-beta-cyclodextrin, phospholipid, Polyvidone K₃₀ and Polysorbate- 80 can make the separated particles fine and difficult to congregate, so it is comparatively stable. By transmission electron microscope, we observe their diameter distribution. The following is the result of the time just after dissolving, 1h and 2h:
The concentration is clinic one: 300mg/1000ml, the diameter distribution as follows:

| Time/Diameter | <50nm | 50~100nm | 100~150nm | 200nm |
|---|---|---|---|---|
| Immediately | 1474 | 20 | 0 | 0 |
| | 1360 | 19 | 0 | 0 |
| 1h | 1754 | 29 | 1 | 0 |
| 2h | 1272 | 41 | 3 | 1 |

After the preparation is stored under room temperature and undergoes the accelerating test, the particle diameter has no tends to enlarge in water. It proves that storage has no influence on the stability of the particle diameter.
4. Security
Zisu (the trade name of Paclitaxel) from the market is compared I in the acute toxicity test with Paclitaxel bought from market: on the same level.
5. Medicine effect comparing test
Three mouse neoplasm cells: Hepatoma (H22), sarcom (S-180), lung cancer (Lewis Lung Cancer, LLC), which is comparing with the Zisu from the market with clinic concentration and dosage, the depressive rate is 27~49.7% higher than the one from market. The test proves that the effect of the product is excellent.

### Clinic application

The products may be dissolved in glucose injection or NaCl injection before administration, the concentration is 300mg/1000ml/3 h at one time, three weeks (or four weeks) for one period of treatment; or the concentration is 100mg/300~500ml at one time, one week for one period of treatment, or big dosage therapy, 400mg/1000ml at one time.

### Industrial Applicability

The nanometer medicines of the invention are inflated multi-hole solid sterile granules or powder, which can use directly for intravenous injection and are targeted, long circulating and release low. They are produced from the matrix of hydroxypropyl-beta-cyclodextrin and phospholipid, hydrophilic organic solvent and water at heating and low pressure, according to the characters of supramolecular chemical micelle, vesicle and other molecules aggregates. As solid preparation for oral administration, it melts quickly and improves the biological utilization. The nanometer medicines adopt safe accessories, general equipment and procedure, which make mass production possible. The paclitaxel for injection of the invention procedure has no polyoxyethelene castor oil, so its reliability and is better than the paclitaxel injection from market.

## Claims

1. A method of preparing solid nanometer medicines, comprising:
A. providing a solution with amphiphiles,
B. adding medicines into the solution with amphiphiles,
C. forming a complex of medicines and amphiphiles, and
D. transforming the complex into solid granules by concentration at a low pressure.

2. The method according to claim 1, wherein the amphiphile is selected from hydroxypropyl-beta-cyclodextrin (HP-β-CD), phospholipids or their combination.

3. The method according to claim 1, wherein the solvent dissolving the amphiphile is selected from hydrophilic solvent, water or their combination.

4. The method according to claim 2, wherein the amphiphile is the combination of hydroxypropyl-beta-cyclodextrin (HP-β-CD) and phospholipids at the weight ratio of 1: 0.05~0.3.

5. The method according to claim 1, wherein the amphiphile is dissolved at 30~100 °C.

6. The method according to claim 5, wherein the amphiphile is dissolved at 60~75 °C.

7. The method according to claim 1, wherein the medicine is at least one of paclitaxel, artemether, dihydroartemisinin, busulfan, nimodipine, nitrendipine, nifedipine, diazepam, cinnarizine, lovastatine and simvastatin.

8. The method according to claim 1, wherein the granule diameter of the complex of the medicines and amphiphiles is less than 300nm with the amphiphile outside and medicines inside.

9. The method according to any one of claims 1 to 7, wherein the solid granules further comprise stabilizer and surfactant.

10. The method according to claim 9, wherein the surfactant is Polysorbate 80.

11. The method according to claim 10, wherein the stabilizer is selected from Polyvidone K₃₀ (PVP K₃₀) or dextran 40, 70.

12. A solid nanometer medicine according to any one of claim 1 to claim 11.

13. The solid nanometer medicine according to claim 12 is paclitaxel.

14. The solid nanometer medicine according to claim 12 or claim 13, wherein the solid nanometer medicine can be used for intravenous injection, intraperitoneal injection, atomizated inhalation and oral administration.

15. An injection prepared with the solid nanometer medicine according to claim 12.

16. The injection according to claim 15, wherein the medicine is paclitaxel.

17. A method of preparing medical complex comprising:
A. providing a solution with the amphiphiles
B. adding medicines into the solution with amphiphiles,
C. forming a complex of medicines and amphiphiles.

18. The method according to claim 17 further comprising transforming the complex into solid sterile granule by concentration in a low pressure.

19. The preparing method according to claim 17 or 18, wherein the amphiphile is selected from hydroxypropyl-beta-cyclodextrin (HP-β-CD), phospholipids or their combination.

20. The preparing method according to claim 17, wherein the solvent dissolving the amphiphiles is selected from water, hydrophilic solvent or their combination.

21. The preparing method according to claim 17, wherein the medicine is at least one of paclitaxel, artemether, dihydroartemisinin, busulfan, nimodipine, nitrendipine, nifedipine, diazepam, cinnarizine, lovastatine and simvastatin.
